# EUROPEAN PATENT APPLICATION

(11) **EP 3 263 103 A1**
(43) Date of publication of application: **03.01.2018**
(21) Application number: 15883501.7
(22) Date of filing: 26.02.2015
(51) Int. Cl.: A61K 31/4152, A61K 31/381, A61K 31/14, A61K 47/00, A61J 3/00, A61P 23/02, A61P 31/00

(54) **PHARMACEUTICAL COMPOSITION FOR TREATMENT OF INFLAMMATORY EAR DISEASES, METHOD FOR PRODUCING SAME AND METHOD FOR TREATMENT USING SAID COMPOSITION**

(71) Applicant: Limited Liability Company "konsortsium-pik", Moscow 125047 (RU)
(72) Inventor: KHMELSHCHIKOV, Yuri Vladimirovich, Moscow 115419 (RU); NOSKOV, Dmitriy Sergeevich, Moskovskaya obl. Reutov 143960 (RU)
(74) Representative: Carvajal y Urquijo, Isabel
(86) International application number: PCT/RU2015/000120
(87) International publication number: WO 2016/137352

(57) **Abstract**

A pharmaceutical composition having an analgesic, anaesthetic and antiseptic action, a method for producing the pharmaceutical composition and a method for the treatment of otitis and other ear injuries. The group of inventions relates to medicine and can be used in the treatment of otitis and other ear injuries. The invention relates to a pharmaceutical composition in the form of ear drops, containing 4 wt.% phenazone or propyphenazone, 2 wt.% articaine and 0.05-0.2 wt.% benzalkonium chloride.

## Description

### Field of the invention

The invention relates to medicine and can be used in the treatment of otitis and other inflammatory ear diseases.

### Background of the invention

Otitis is a disease that is related with an inflammatory process within the ear. Human ear includes outer ear, middle ear and inner ear.

In parallel with pathological process localization, outer otitis, middle otitis and internal (inner) otitis can occur.

Outer otitis can appear in a confined or diffuse form. Ear canal furuncle is an example of confined outer otitis. Diffuse outer otitis is represented by a large group of bacterial, viral or fungal inflammatory diseases.

Middle ear diseases occur in human belonging to various age groups and are clinically and socially significant. A variety of pathogenetic mechanisms of these diseases is determined by the particular anatomic and physiological features of the middle ear, specific ethiology, immune status etc. Generally, the definition of nosology reflects predominant disease process in one or another area of the middle ear. Acute middle otitis commonly corresponds to the prevalent inflammation in the tympanic cavity, acute inflammation of the auditory tube is called eustachitis, inflammation of the mastoid processus is named mastoiditis. The course of the middle otitis can be acute or chronic, the inflammation can occur in the catarrhal, serous or purulent variants (Palchun V.T. et al. / Otorhinolaryngology: handbook. The 2nd ed., ext. and suppl. - M.: GEOTAR-Media, 2011 - 656 pp.: fig.; ISBN 978-5-9704-1804-8).

One of the methods of treatment of inflammatory ear diseases includes ear drops instillation. Conventional ear drops are generally based on the aminoglycoside antibacterial agents (Sofradex, Garazone, Anauran). The main groups of microbes, that induce inflammatory ear diseases, are sensitive to aminoglycosides. However, potential ototoxicity of aminoglycosides restricts their use by the outer otitis and middle otitis without perforation (Baliasinskaia G.L. Ear drops otofa and polydex for treatment of acute external otit media in children // Vestnik otorinolaringologii. 2003. No.3. p. 53-54).

The patent application WO2009142719, MASSACHUSETTS INSTITUTE OF TECHNOLOGY et al., A61K 8/00, 8/18, published on 26 of November, 2009 on 83 pages (D1) describes the pharmaceutical composition intended to treat the inflammatory ear diseases and containing therapeutic components that can include anaesthetic agent, e.g., articaine, or anti-inflammatory medication, e.g., phenazone.

The patent claim CN101584689 B, NAVY MEDICINE RESEARCH INSTITUTE OF PLA, A61K31/167, 31/4152, A61P27/16, 29/00, published on 16 March 2011 on 9 pages (D2), describes the pharmaceutical composition intended to treat otitis and containing phenazone and lidocaine.

The technical solution described in D2, can be adopted as a proximal analogue.

The components of the claimed pharmaceutical composition are first proposed by the authors. Combined use of articaine and propyphenazone (as well as combined use of articaine, propyphenazone and benzalkonium chloride) to produce the pharmaceutical composition intended for external or local application, are proposed by the authors for the first time.

### Summary of the invention

In the context of the above facts, the purpose of the present invention consists in the creation of a new pharmaceutical composition providing treatment of otitis.

The technical result of the present invention consists in the improved efficacy (enforced clinical effect) of the treatment of otitis and providing of efficacious and stable pharmaceutical composition aimed to be used in the treatment of otitis.

The embodiments of the present invention include pharmaceutical composition aimed to treat otitis and containing at least phenazone (or propyphenazone) and articaine, as well as benzalkonium chloride.

The other embodiment of this invention includes the method of treatment of otitis and the method of production of pharmaceutical composition intended to treat otitis.

### Summary of Fig.s

In Fig. 1, the photograph of the experimental rabbit ear following scarification, prior to administration of Formulation 1, is presented.
In Fig. 2, the photograph of the experimental rabbit ear following scarification, 1 day after the first administration of Formulation 1, is presented.
In Fig. 3, the photograph of the experimental rabbit ear following scarification, 2 days after the first administration of Formulation 1, is presented.
In Fig. 4, the photograph of the experimental rabbit ear following scarification, 3 days after the first administration of Formulation 1, is presented.
In Fig. 5, the photograph of the experimental rabbit ear following scarification, prior to administration of Formulation 2, is presented.
In Fig. 6, the photograph of the experimental rabbit ear following scarification, 1 day after the first administration of Formulation 2, is presented.
In Fig. 7, the photograph of the experimental rabbit ear following scarification, 2 days after the first administration of Formulation 2, is presented.
In Fig. 8, the photograph of the experimental rabbit ear following scarification, 3 days after the first administration of Formulation 2, is presented.
In Fig. 9, the photograph of the experimental rabbit ear following scarification, prior to administration of Formulation 3, is presented.
In Fig. 10, the photograph of the experimental rabbit ear following scarification, 1 day after the first administration of Formulation 3, is presented.
In Fig. 11, the photograph of the experimental rabbit ear following scarification, 2 days after the first administration of Formulation 3, is presented.
In Fig. 12, the photograph of the experimental rabbit ear following scarification, 3 days after the first administration of Formulation 3, is presented.
In Fig. 13, the photograph of the control rabbit ear following scarification is presented.
In Fig. 14, the photograph of the control rabbit ear following scarification 1 day later is presented.
In Fig. 15, the photograph of the control rabbit ear following scarification 2 days later is presented.
In Fig. 16, the photograph of the control rabbit ear following scarification 3 days later is presented.

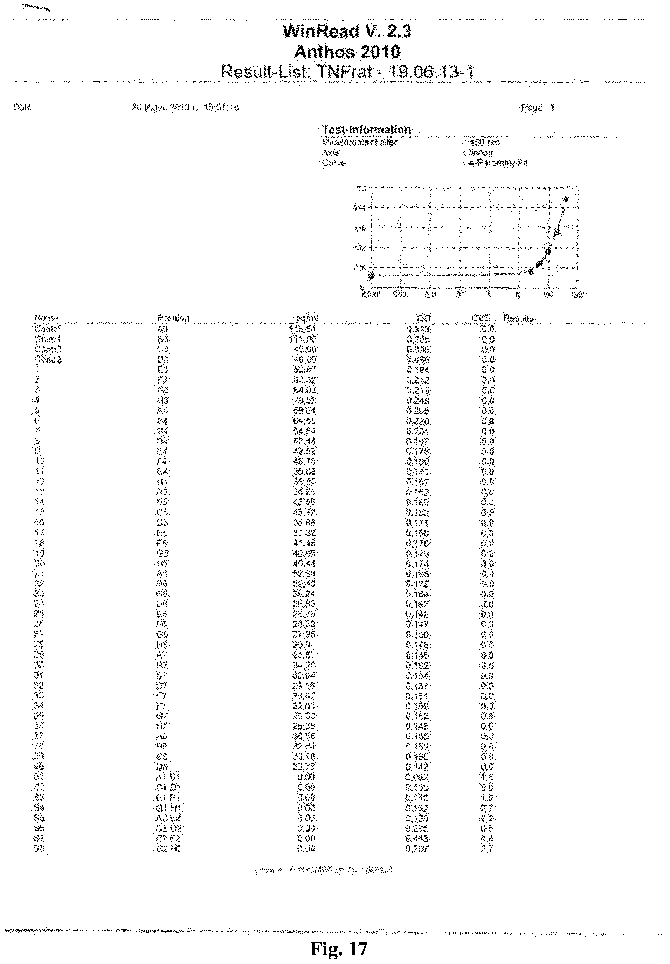

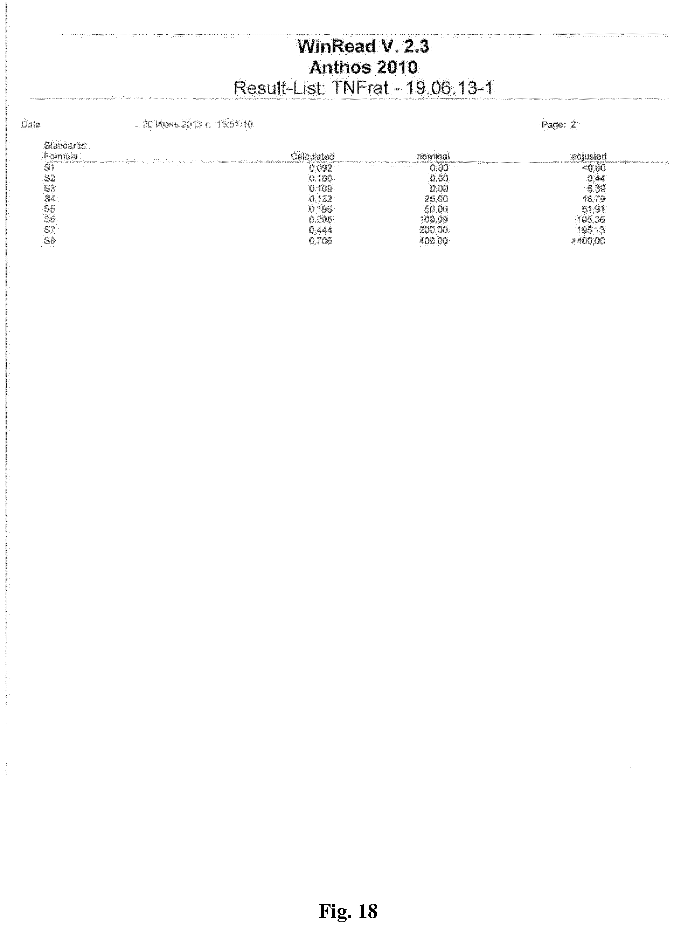

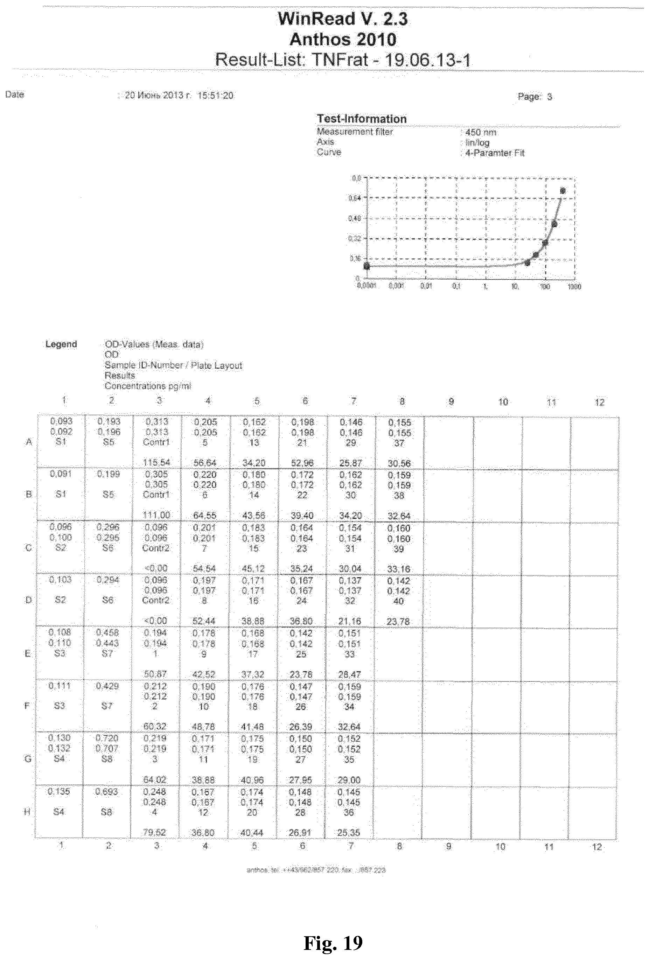

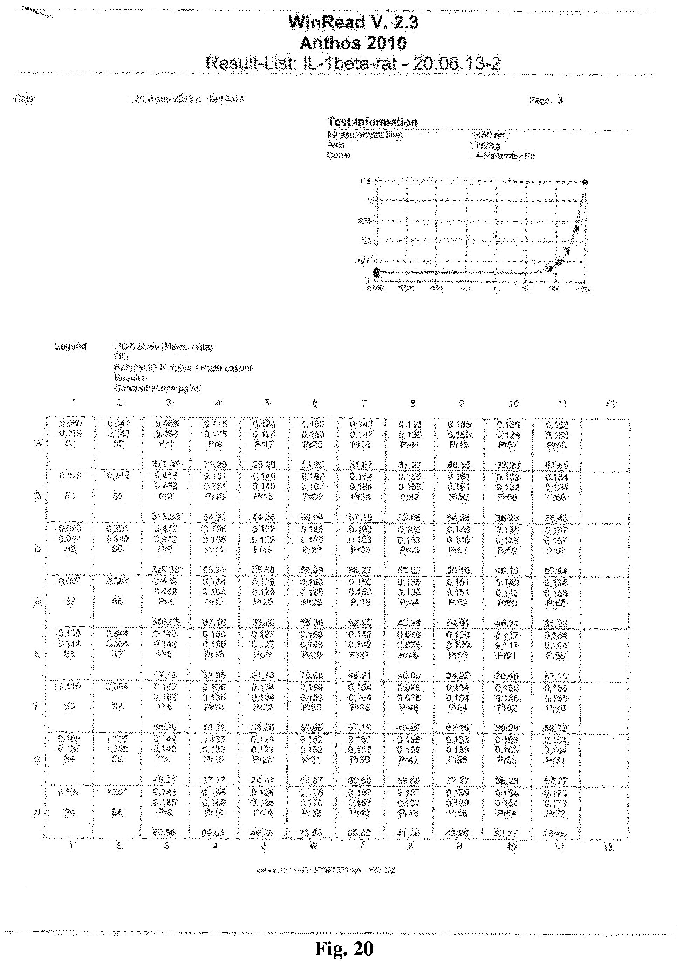

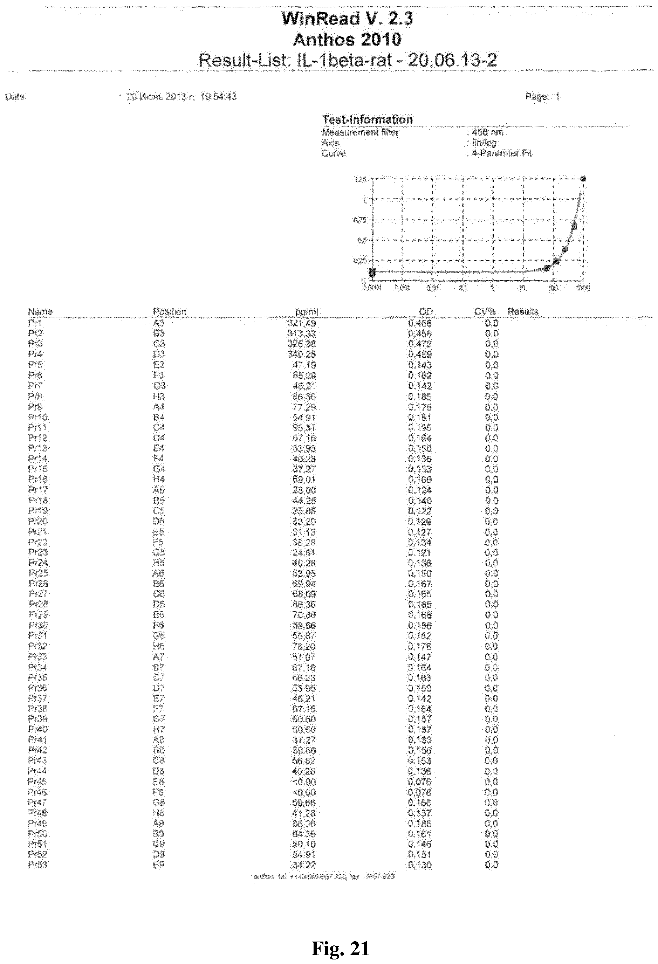

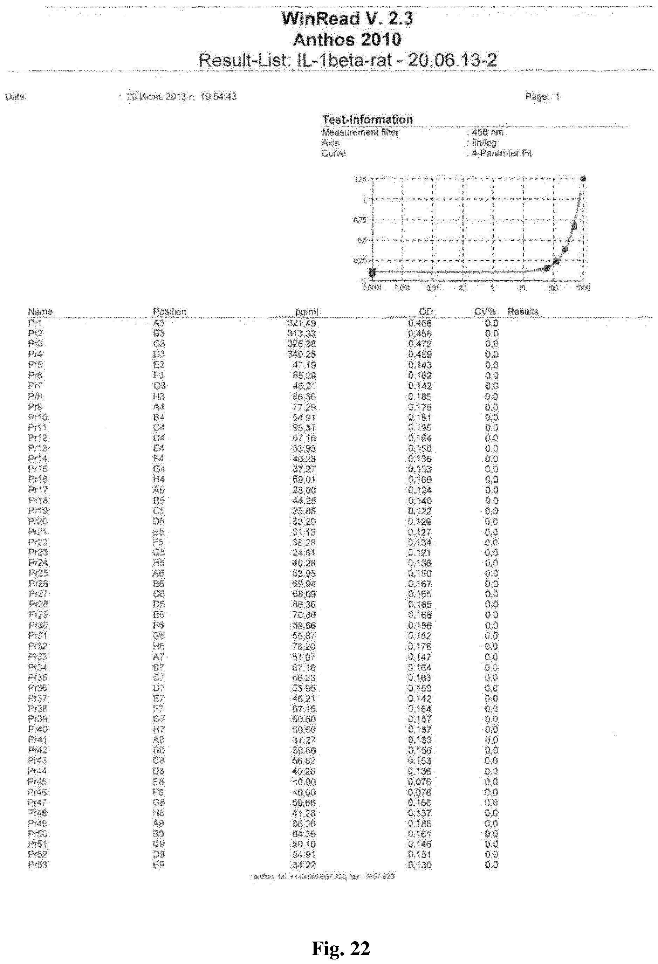

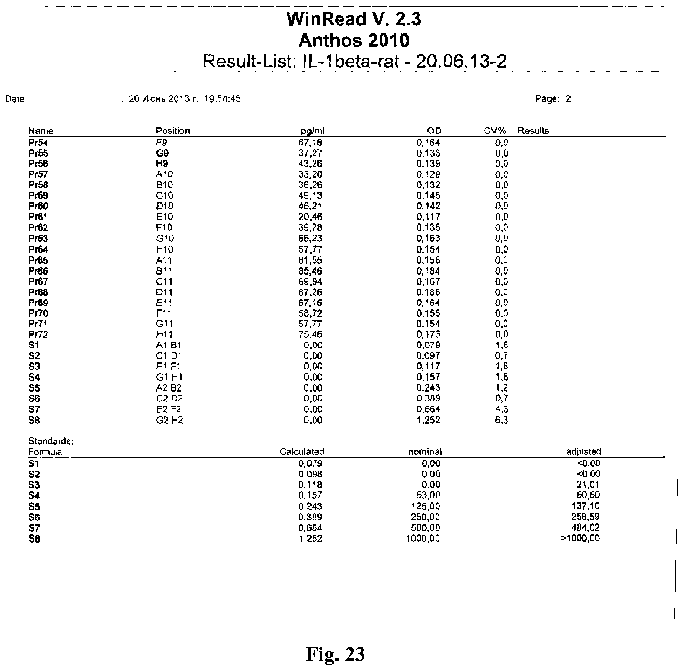

In Figs. 17-23, baseline data of immunoassay for cytokines IL-1β and TNF-α are presented.
In Fig. 24, TNF-α level in the supernatant of activated macrophages (MPh) after 6-hour incubation dependent on the level of benzalkonium chloride, is presented.
In Fig. 25, TNF-α level in the supernatant of activated macrophages (MPh) after 24-hour incubation dependent on the level of benzalkonium chloride, is presented.
In Fig. 26, the effect on production of IL-1β in the supernatant of activated MPh after 6-hour incubation dependent on the level of benzalkonium chloride, is presented.
In Fig. 27, the effect on production of IL-1β in the supernatant of activated MPh after 24-hour incubation dependent on the level of benzalkonium chloride, is presented.

### Embodiment of invention

Phenazone (trade name: Antipyrine) is a medicinal agent, analgesic and antipyretic belonging to pyrazolone group.

### Structural formula of phenazone:

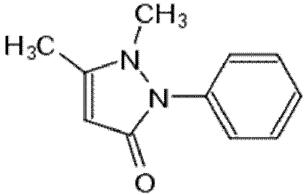

### IUPAC nomenclature: 1,2-dihydro- 1,5-dimethyl- 2-phenyl- 3H-pyrazol- 3-one

Pharmaceutical form: colourless grains or white granular powder, odourless, slightly bitter. Very easily soluble in water (1:1), easily soluble in the ethanol. The solutions (pH 6.0-7.5) are sterilized at +120°C for 20 minutes.

Phenazone was first synthesized by Ludwig Knorr (Höhst) in 1883.

Phenazone was one of the first synthetic analgesics, pyrazolone derivatives, used in medicine (1884). Following the appearance of other analgesics, its use became relatively rare. Currently, phenazone is not widely applied.

Like other pyrazolone derivatives, phenazone provides pain-releasing, antipyretic and some degree of anti-inflammatory effect. Analgesic and antipyretic activity of these drugs is close to that of acetylsalicylic acid derivatives. Pyrazolone derivatives reduce capillary permeability and inhibit inflammatory response.

Phenazone provides moderate analgesic, antipyretic and antiinflammatory effects. Local application leads to a certain degree of haemostatic effect. It is used in neuralgia, cold.

Propyphenazone is an analgesic and antipyretic of pyrazolone group, phenazone derivative, which exerts the similar pain-releasing and antipyretic effects.

### Structural formula of propyphenazone:

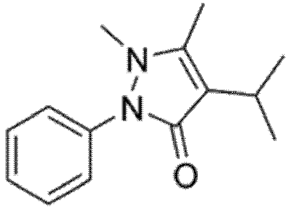

### IUPAC nomenclature: 1,5-Dimethyl-2-phenyl-4-propan-2-yl-pyrazol-3-one.

Upon oral administration, the drug exerts significant analgesic and antipyretic effects, its anti-inflammatory activity being weak, similar to other pyrazolones. It is readily absorbed in the digestive tract, maximal blood level can be achieved within 30 minutes following oral administration.

Propyphenazone is the most safe compared to other pyrazolone derivatives. In particular, its administration was not associated with agranulocytosis.

Moreover, it should be noted that in view of prior art, propyphenazone was not used as active component of pharmaceutical composition for external or local administration.

Articaine (trade name - Ultracaine) is a highly active anaesthetic medicinal agent.

In particular, within the present invention the pharmaceutical composition including articaine hydrochloride is described.

### Structural formula of articaine:

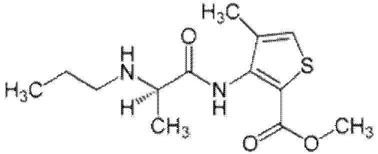

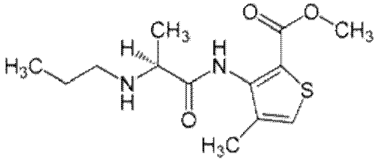

### IUPAC nomenclature:

### (RS)-Methyl 4-methyl-3-(2-propylaminopropanoylamino)thiophene-2-carboxylate.

Articaine is a local anaesthetic used for infiltration and regional anaesthesia. The drug exerts significant analgesic effects, 2-fold more potent vs. lidocaine and 6-fold vs. procaine. The anaesthetic penetrates the membrane of the nervous fibers, releasing lipophilic base due to the hydrolysis in the weakly alkaline tissue environment (this effect decreases in the acidic environment). Therefore, ultracaine come into interaction with nerve receptors, inhibits depolarization Na+ intracellular influx and impulse conduction along the nerve fiber. Anaesthesia begins immediately following drug administration and persists up to 1-5 hours.

The drug was first launched into dental practice in 1978. Its active substance is represented by articaine hydrochloride (methyl ether of 4-methyl-3[2-propylaminopropionamide]-2-thyophecarboxylic acid) + epinephrine hydrotartrate (epinephrine).

Benzalkonium chloride is an antiseptic agent with fungicide, antiprotozoan, local contraceptive (spermicidal) effects, antiviral (Herpes simplex) inactivating effect.

### Structural formula of benzalkonium chloride:

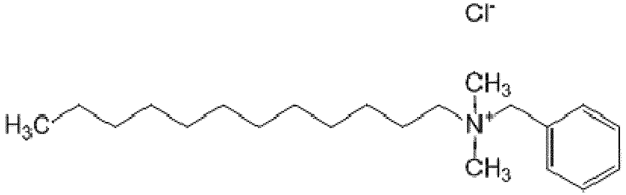

### IUPAC nomenclature: Alkyldimethylbenzylammonium chloride.

It exerts bactericidal effects on Staphylococcus, Streptococcus, gram-negative bacteria (E. coli and Pseudomonas aeruginosa, Proteus, Klebsiella etc.), anaerobes, fungi, including moulds and yeasts, and viruses. It is active against bacterial stains resistant to antibacterials and other chemotherapeutic agents; inhibits staphylococcal plasmocoagulase and hyaluronidase. The drugs prevents from secondary wound infection associated with hospital microbial stains. Its spermicidal effect is related with its ability to hurt spermatozoa membranes (first flagellum, then head membrane), leading to the inability of fertilization due to the spermatozoa impairment. The effect develops within 8 to 10 minutes (tablets), 5 minutes (vaginal suppositoria), 3 minutes (cream) or immediately following vaginal insertion (tampon). It is active *in vitro* against Neisseria gonorrhoeae, Chlamydia spp., Trichomonas vaginalis, Human herpesvirus 2, Staphylococcus aureus. The drug does not affect Mycoplasma spp. and is weakly active against Gardnerella vaginalis, Candida albicans, Haemophilus ducreyi and Treponema pallidum. *In vivo,* it provides a certain activity against several sexually transmitted diseases. It does not influence on the normal vaginal microflora (incl. Doderlein bacilli) or hormonal cycles.

Propylene glycol is used as an excipient and main solvent of propyphenazone substance (in combination with glycerol). Moreover, taking into account the absence of preservatives in the claimed compositions, it can act as preservative agent due to its bactericidal effects.

### Structural formula of propylene glycol:

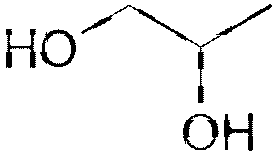

### IUPAC nomenclature: Propane-1,2-diol.

Glycerol is a co-solvent excipient, acting as water substitute, decreasing the amount of the solvent (water) that does not dissolve the active substance of propyphenazone, thus providing the stability of composition during storage. Due to its viscosity, it increases total viscosity of composition, contributing to the improvement of its rheological properties and drop dispensing during administration.

### Structural formula of glycerol:

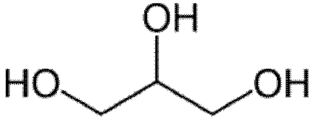

### IUPAC nomenclature: propane-1,2,3-triol.

The main embodiment of the present invention, respectively, consists in the pharmaceutical composition with analgesic, anaesthetic, antiseptic and anti-inflammatory effects, which can be used in treatment of otitis, i.e. outer and middle otitis, and of other inflammatory ear diseases, containing, at least: phenazone or propyphenazone as analgesic, and benzalkonium chloride as antiseptic and anti-inflammatory agent.

Another embodiment of the present invention provides for the pharmaceutical composition with analgesic, anaesthetic and bactericidal effects, containing, at least: phenazone or propyphenazone as analgesic, 4% w/w; articaine as anaesthetic, 2% w/w, and benzalkonium chloride as antiseptic, 0.05% to 0.2% w/w.

Another embodiment of the present invention provides for the pharmaceutical composition with analgesic, anaesthetic and bactericidal effects, containing, at least: phenazone or propyphenazone as analgesic, 4% w/w; articaine as anaesthetic, 2% w/w; benzalkonium chloride as antiseptic, i.e., 0.05% to 0.2% w/w; and optionally containing additionally propylene glycol as excipient, i.e., 44% to 60% w/w; and containing additionally glycerol as excipient, i.e., 20% to 30% w/w.

Another embodiment of the present invention provides for the pharmaceutical composition in any of above embodiments of invention, with a particular characteristic of presentation as ear drops.

Another embodiment of the present invention provides for the method of production of pharmaceutical composition containing, at least, propyphenazone, articaine and benzalkonium chloride, which includes:
propyphenazone dissolution in propylene glycol at 30-45°C until complete dissolution and articaine and benzalkonium chloride dissolution in water at 30-45°C while stirring until complete dissolution;
combining of the obtained solutions of propyphenazone in propylene glycol and articaine, benzalkonium chloride and water while stirring until homogeneity of the mixed solution;
adding of glycerol to the mixed solution while stirring until homogeneity of the solution obtained through adding of glycerol;
gradual adding of water up to target volume while stirring.

Another embodiment of the present invention provides for the above-described method of production of pharmaceutical composition, containing, at least, propyphenazone, articaine and benzalkonium chloride, characterized in the intense agitation.

Another embodiment of the present invention provides for the above method of production of pharmaceutical composition, containing, at least, propyphenazone, articaine and benzalkonium chloride, characterized in that the combining of the obtained solution of propyphenazone and propylene glycol with the obtained solution of articaine, benzalkonium and water is performed while stirring for not more than 30 minutes.

Another embodiment of the present invention provides for the above method of production of pharmaceutical composition, containing, at least, propyphenazone, articaine and benzalkonium chloride, characterized in that the adding of glycerol to the solution obtained through mixing is performed while stirring for not more than 30 minutes.

Another embodiment of the present invention provides for the above method of production of pharmaceutical composition, containing, at least, propyphenazone, articaine and benzalkonium chloride, characterized in that following adding of water up to target volume, the filtration that comprises sieve, comprising nylon sieve.

Another embodiment of the present invention provides for the method of production of pharmaceutical composition containing, at least, phenazone, articaine and benzalkonium chloride, which includes:
dissolution of phenazone, articaine and benzalkonium chloride in water at 30-45°C while stirring mixing until complete dissolution;
adding of glycerol to the mixed solution while stirring until homogeneity of the solution obtained through adding of glycerol;
gradual adding of water up to target volume while stirring.

Another embodiment of the present invention provides for the above described method of production of pharmaceutical composition, containing, at least, phenazone, articaine and benzalkonium chloride, characterized in the intense agitation. Another embodiment of the present invention provides for the above method of production of pharmaceutical composition, containing, at least, phenazone, articaine and benzalkonium chloride, characterized in that the adding of glycerol to the solution obtained through stirring is performed while stirring for not more than 30 minutes.

Another embodiment of the present invention provides for the above method of production of pharmaceutical composition, containing, at least, phenazone, articaine and benzalkonium chloride, characterized in that following adding of water up to target volume, the filtration is performed that comprises sieve, comprising nylon sieve.

Another embodiment of the present invention provides for the method of treatment of otitis, including the instillation of the pharmaceutical composition in any of the above aspects in the form of ear drops into the ear of patient with an inflammatory ear disorder.

Another embodiment of the present invention provides for the method of treatment of otitis, including the instillation of the pharmaceutical composition in any of the above aspects in the form of ear drops into the ear of patient with an inflammatory ear disorder, wherein the inflammatory disorder comprises outer or middle otitis.

### Detailed description of embodiment

Antiseptic and anti-inflammatory activity of the pharmaceutical composition.

Antiseptic and anti-inflammatory effects were verified for the following formulations of the pharmaceutical composition:
Formulation 1 - phenazone 4% w/w, articaine 2% w/w, benzalkonium chloride 0.2% w/w;
Formulation 2 - phenazone 4% w/w, articaine 2% w/w, benzalkonium chloride 0.1% w/w;
Formulation 3 - phenazone 4% w/w, articaine 2% w/w, benzalkonium chloride 0.05% w/w;
Formulation 4 - propyphenazone 4% w/w, articaine 2% w/w, benzalkonium chloride 0.2% w/w;
Formulation 5 - propyphenazone 4% w/w, articaine 2% w/w, benzalkonium chloride 0.1% w/w;
Formulation 6 - propyphenazone 4% w/w, articaine 2% w/w, benzalkonium chloride 0.05% w/w.

Antiseptic and anti-inflammatory effects were tested in animal for the formulations 1 to 3.

Anti-inflammatory and antiseptic effects of three formulations of the ear drops were assessed from the wound-healing effects in rabbits with skin impairment and from the histomorphology of the wound surface imprint smears. A significant effect was observed in the type and duration of wound healing, At this, according to the decrease of the total leukocyte level on the wound secretion, the sample ear drops exert anti-inflammatory effect.

The main indication for the ear drops under development includes otitis. There is no adequate model for this condition in animal, therefore, anti-inflammatory effects of three samples of ear drops were studied in the model of scarified wounds in rabbits. The main criteria of drug effects assessment included type and duration of wound healing, as well as morphological features of wound secretion.

### Testing system

The tests were conducted in male chinchilla rabbits 2.5 to 3.0 kg. The animals were purchased from the branch Andreevka of the State Institution Scientific Center "Biomedical technologies" of the Russian Academy of Medical Sciences; they were kept at the vivarium in accordance with the sanitary rules, with standard meal including dry granules.

Three experimental groups were formed, 4 rabbits per each ear drops formulation:
Group 1 - Formulation 1
Group 2 - Formulation 2
Group 3 - Formulation 3

### Description of the experiment

Scarified skin wounds were reproduced as follows: Several superficial scratches were made with the use of scalpel on the outer ear (taking into account the ear drops destination, in close proximity the ear canal) of each rabbit at the surface 1x1 cm. Approximately 1 hour later, each experimental rabbit received a certain formulation of ear drops onto the wound surface of the ear (thereafter -experimental ear), 2 to 3 ear drops. Ear drops applications were performed on two occasions 24 hours apart until the solid slough formation.

Wound surface of the opposite left ear, which was not subjected to treatment, served as control condition (thereafter - control ear)

During the whole experiment, general condition and behaviour of the experimental rabbits were monitored.

Clinical condition of the wounds and adjacent tissues, duration of the slough formation and complete wound healing were assessed daily, for 7 days in total. Immediately following scarification and 24 hours after the treatment, the imprint smears were taken from the wound surface of the experimental ear of each rabbit; within the same period, the imprint smears were taken from the control ear wounds. The smears were stained with azur-eosin. Microspecimens were assessed using microscope Micros (Austria) with 90x10 magnification.

### Results

Immediately after the scarification and during further period of observation (7 days), the overall condition and behaviour of the rabbits did not undergo substantial changes. All the animals readily consumed food and water.

The wound surface was monitored daily. The time course of these observations is provided below.

Following application of three sample formulations on the wound surfaces the signs of epithelization appeared as the peripheral edge around the wounds. In the center of the wound, a thin, dry and colourless skin was seen. No purulent or blood discharge was observed. The start of slough formation was recorded. No erythema, swelling or induration were present in the area around the wound.

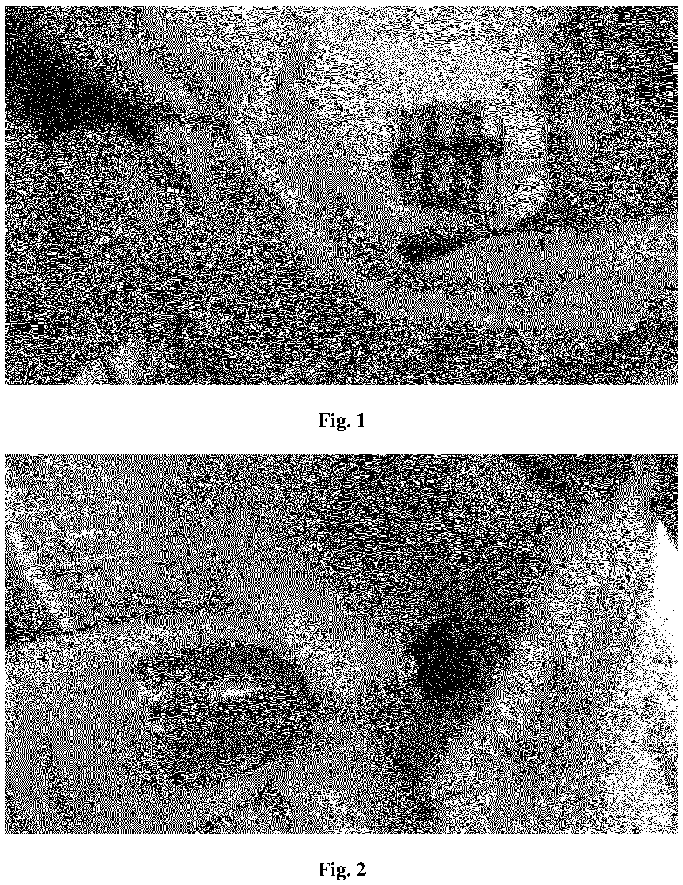

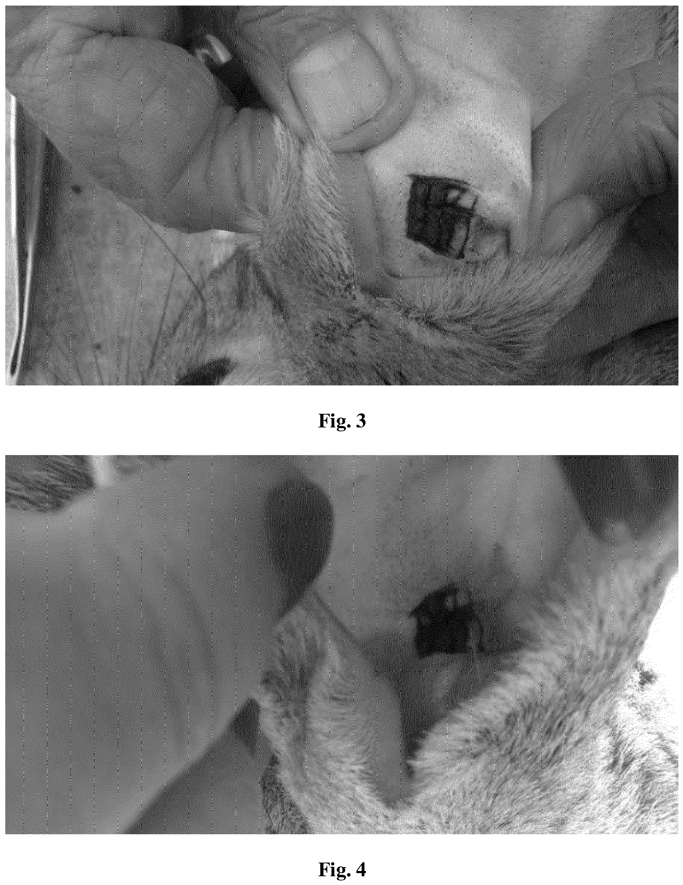

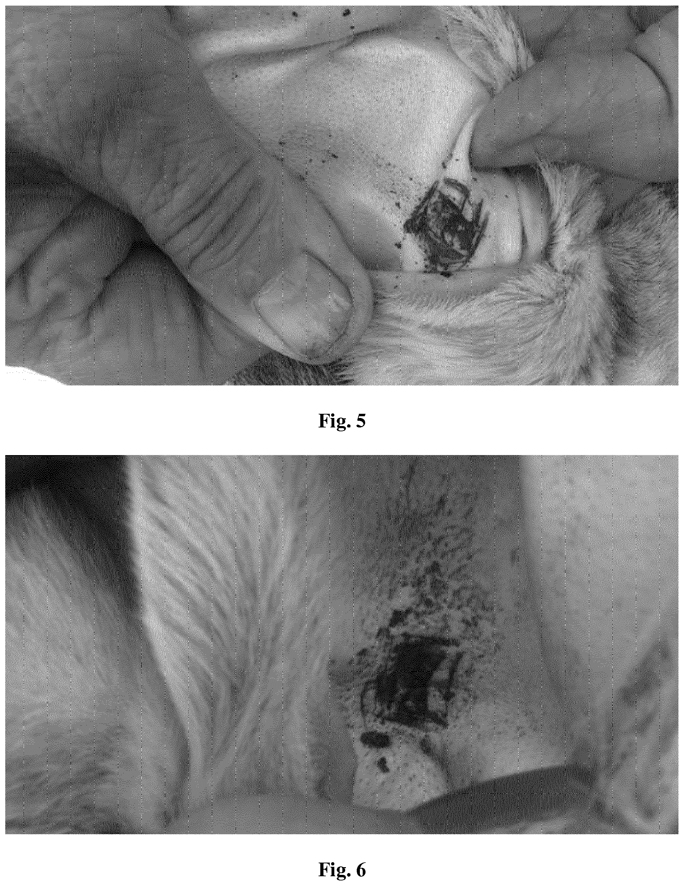

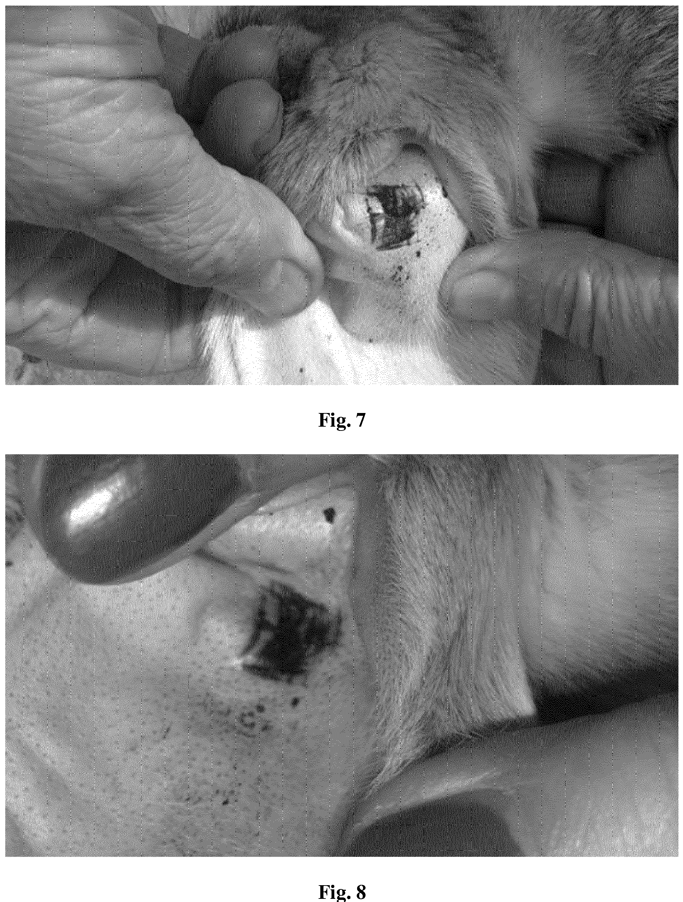

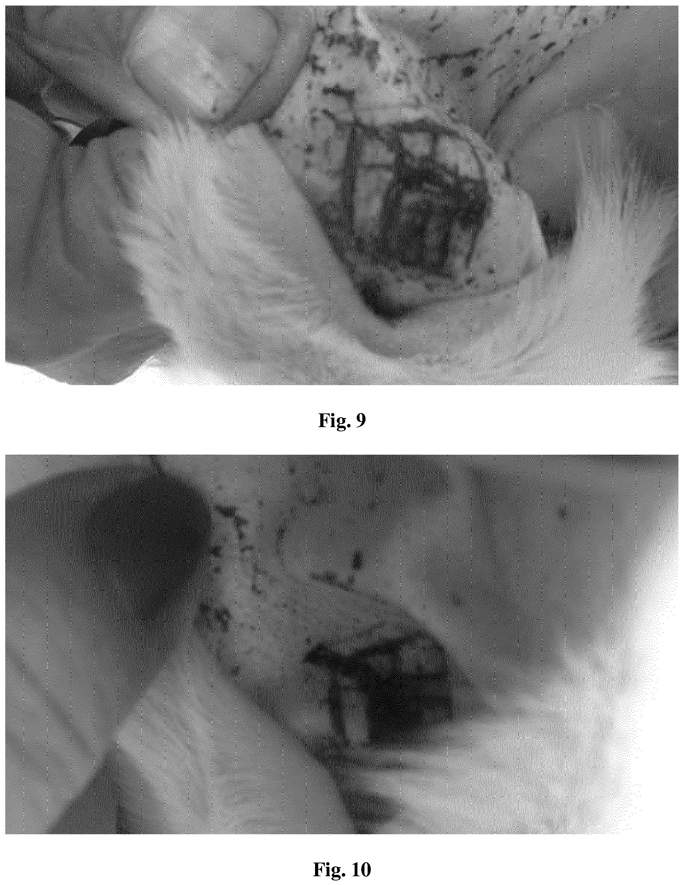

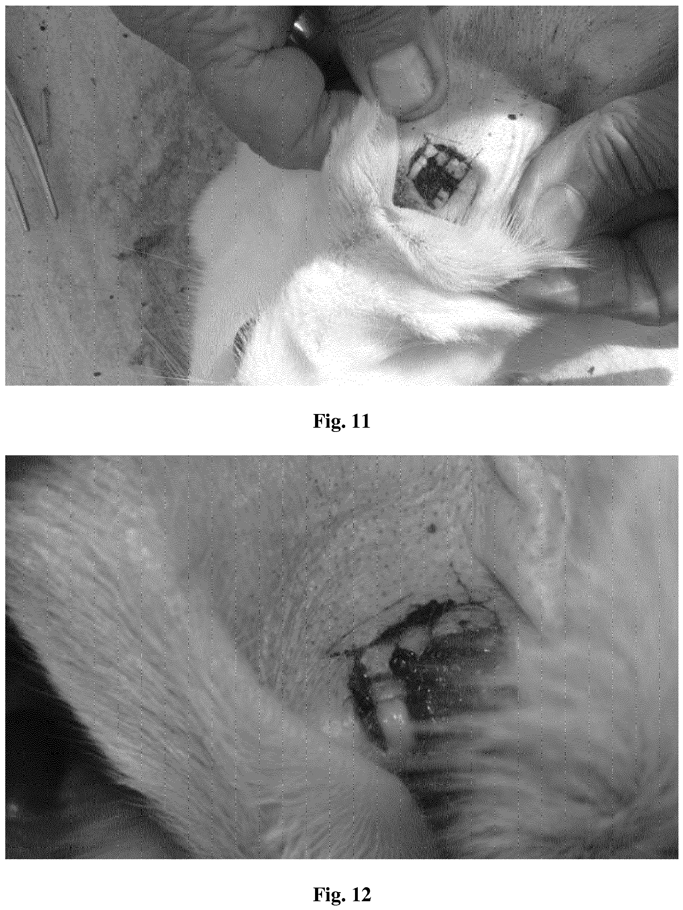

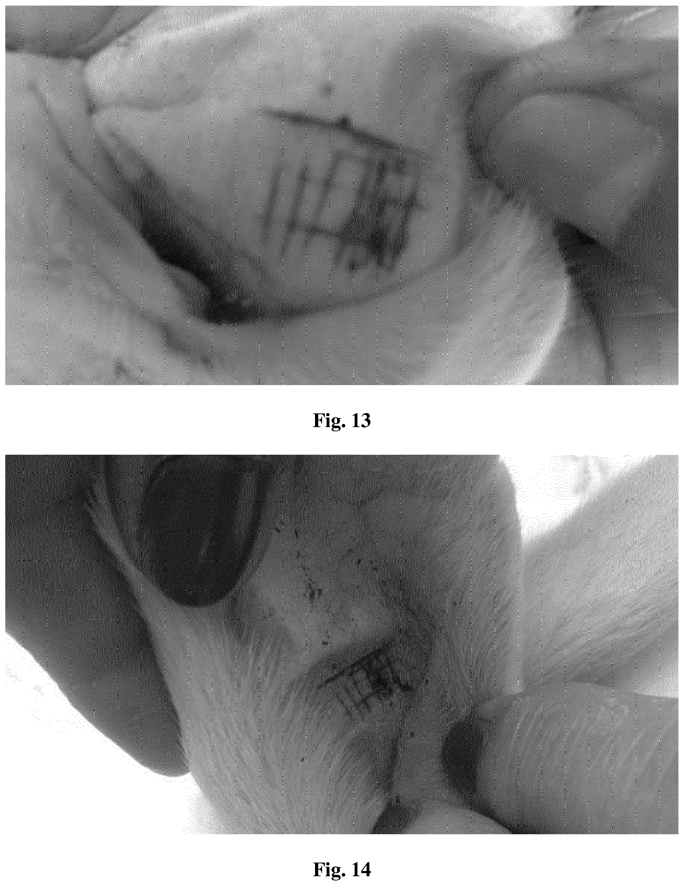

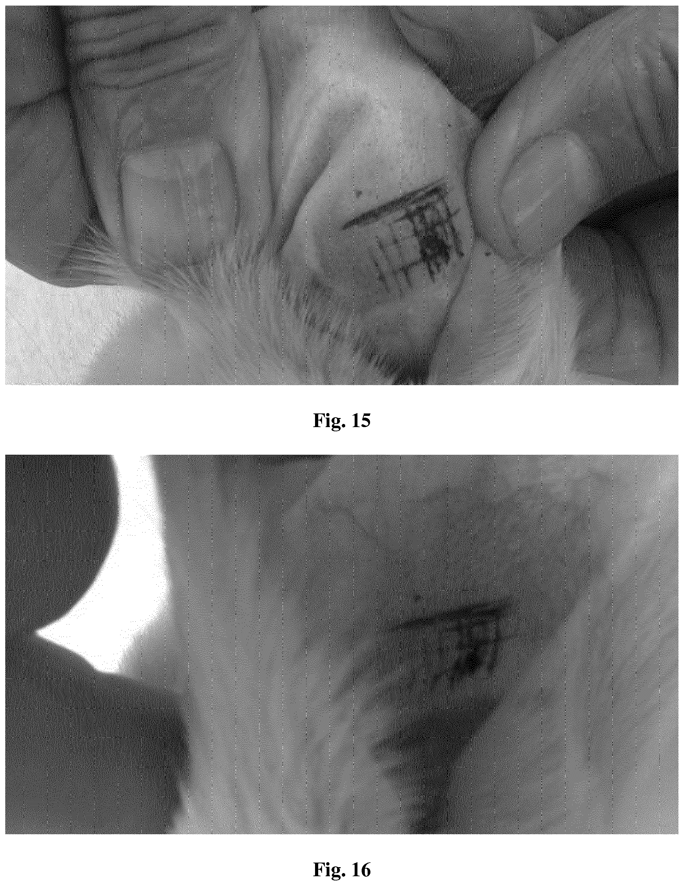

Within 48 hours, the complete formation of the slough was only observed in the experimental ears. The slough was clean, smooth, solid and dry. No wound discharge was observed in any animal. The skin around the wounds was smooth, flat, clean, without erythema or swelling. No signs of inflammation were observed (see Figs. 1-12). On Day 3, the formation of the slough in the control ear was not completed (see. Figs. 13-16).

Further wound healing was present thereafter. In the control group, the complete recovery of skin impairment occurred by Day 7. In the experiment group, the complete recovery of skin impairment occurred by Day 4-5.

Therefore, formulations 1-3 provide significant antiseptic and anti-inflammatory activity, leading to faster slough formation and recovery of the experimental ear.

### Materials and methods of antiinflammatory testing of formulations 1 to 3 in vitro

Production of rat sensitized peritoneal macrophages (MPh) culture

Active MPh were harvested of rat peritoneal exudate 4 days after the intraperitoneal injection of 5 mL of horse serum. The peritoneal cavity was rinsed with PBS (PanEco, Russia) and the resulting suspension collected into the centrifuge tube (CORNING, 50 mL). Following centrifugation (Eppendorf, 5702RH, Germany) (5 min, 200 rpm), supernatant was removed, the pellet resuspended in the growth medium RPMI-1640 (GIBCO) with 10% FBS (GIBCO), L-glutamine and antifungal antibacterial agent (GIBCO) in the conventional concentration. Mononuclear cells were counted in Goryaev's chamber at Olympus microscope (Olympus CK 40, Japan), thereafter cell suspension 1.6 x 10⁶ cells/mL was prepared. One mL of mononuclear suspension was introduced per each well of 24-well plate (CORNING) and incubated in moist CO2 atmosphere (New Brunswick, Galaxy 170R) at t=37°C and 5% CO2.

To perform the experiment, the harvested MPh were inoculated to two 24-well plates. After 2-hour incubation non-adhesive cells were removed, and the wells rinsed with a warm Hanks solution (PanEco, Russia). Adhesive MPh accounted up to 50-60% of the total number of mononuclear cells inoculated.

Preparation of the study drugs. The effect of the drugs on the sensitized MPh was studied in three dilutions. For this purpose, the formulations 1 to 3 were prepared at the growth medium RPMI-1640 (GIBCO) with addition of 1% FBS, L-Glutamine and antibacterial/antifungal agent, in the concentrations indicated.

Up to 500 mcL/well of sterile solutions of formulations 1 to 3 were introduced. The control wells were subdivided into two groups: K1 and K2, four wells per each group. In the first group (K1) up to 500 mcL of the initial growth medium RPMI-1649 with 1% FBS without study drugs were introduced. In the second group (K2), the growth medium contained phenazone and articaine.

Supernatant samples for immunoassay were obtained in 4 repeats, 6 hours apart and 24 hours after introduction of the study drugs. Prior to freezing, supernatant was centrifugated (2 minutes, 10,000 rpm) and stored in aliquotes at -70°C until immunoassay (EIA).

### Immunoassay

The quantitative assessment of soluble cytokines IL-1β and TNF-α in the supernatant of activated MPh was performed on the basis of enzyme-linked immunoassay. The experiments were performed with the standard EIA reagent sets "Quantikine ELISA Rat TNF- α" (Catalog Number RTA00) 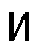 "Quantikine ELISA Rat IL-1β" (Catalog Number RLB00), manufacturer R&D Systems, United Kingdom. The analysis was conducted in accordance with the conventional protocol. Optical density was measured with the use of reader-spectrophotometer Anthos 2010 (Austria) at 450 nm. The baseline EIA data for both cytokines are provided in Fig.NºNº 17-23.

### Results

The study explored the influence of the formulations 1 to 3 on the production of IL-1β and TNF-α, associated with activated MPh in the *in vitro* model. In the course of the experiment, fluoroscopic control of MPh culture was performed. After 6 hours and 24 hours of culturing, the visual inspection did not reveal any morphological differences of the study wells vs. control ones. In any wells, the cells were similarly flattened, no detritus was present in the growth media. Therefore, the study drugs did not induce any cytotoxic effects on the cell culture following 24-hour incubation.

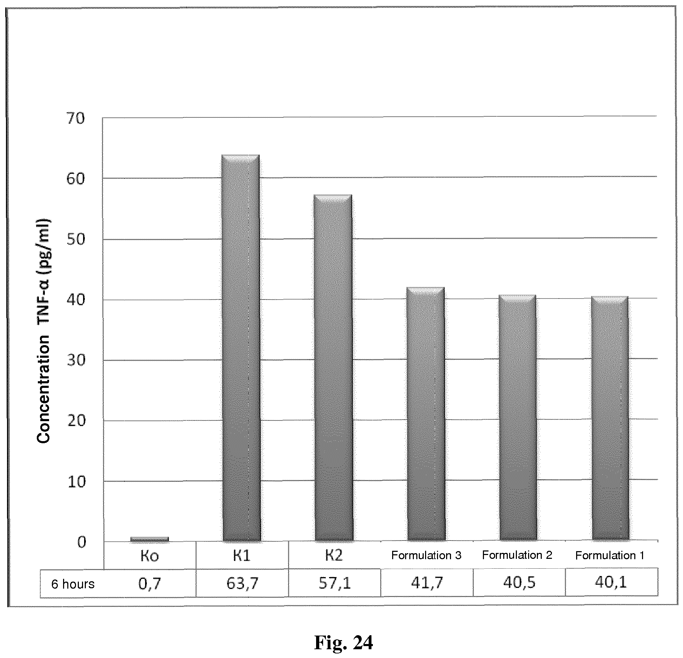

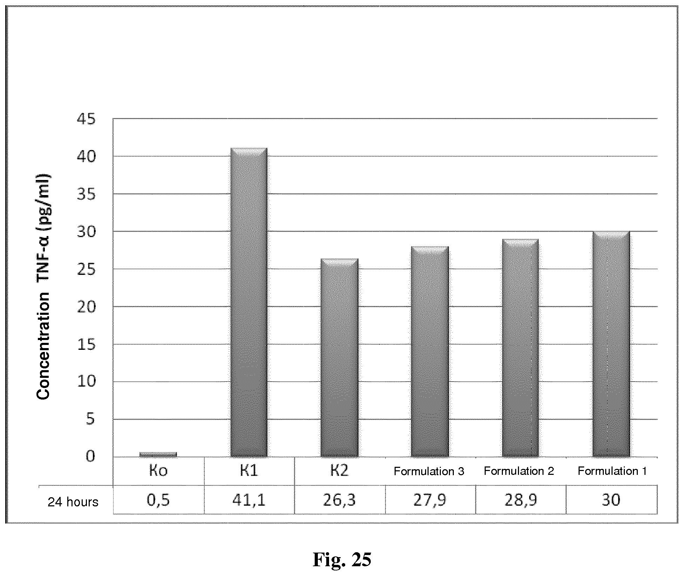

Figs. 24 and 25 show the level of TNF-α in the supernatant of activated MPh after 6-hour and 24-hour incubation dependent on the level of benzalkonium chloride. Co. = control (non-conditioned RPMI-1640 medium with 1% PBS). After 6 hours, TNF-α level decreased by a mean of 30% compared to the control groups K1 and K2. At this, formulations 1 to 3 similarly inhibited the synthesis of TNF-α in the activated macrophages. This trend persisted by the end of the 24-hour period, at the endpoint of the study. Therefore, it can be concluded that the study formulations inhibited the 24-hour synthesis of TNF-α by the activated MPh at the same degree.

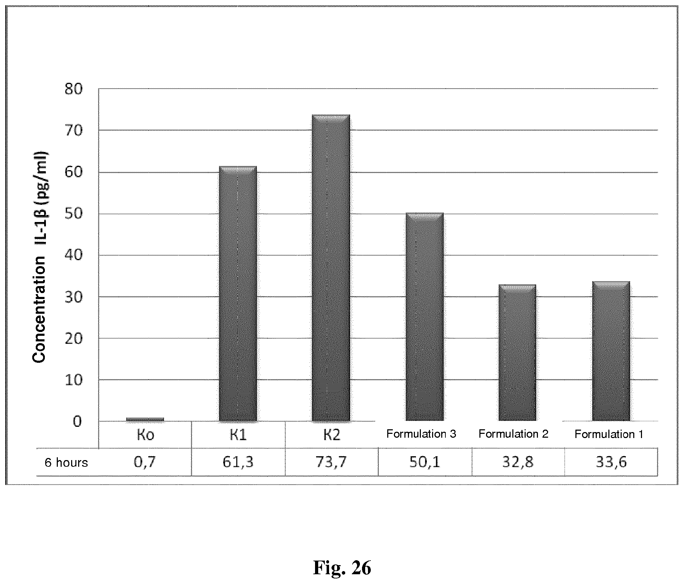

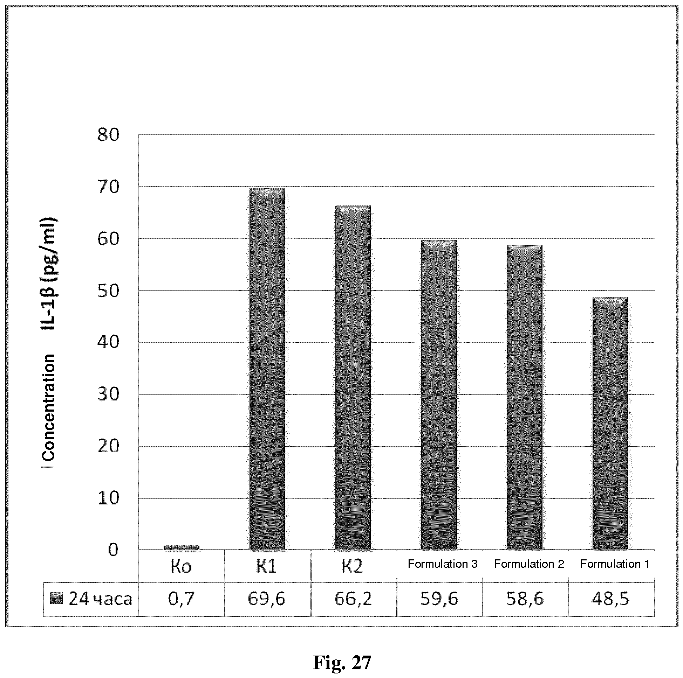

Figs. 26 and 27 show the effect of the study drugs on the synthesis of IL-1β in the supernatants of activated MPh. The level of IL-1β tended to decrease by the end of 6-hour incubation. Due to the effect of the formulations 1 and 2, the level of IL-1β decreased approximately 2-fold vs. control groups K1 and K2. The formulation 3 inhibited IL-1β synthesis in less significant degree. By the end of 24 hours, the inhibiting effect of the study drugs weakened.

Thus, the study of anti-inflammatory activity of the formulations 1 to 3 related with the activated MPh demonstrated the most pronounced anti-inflammatory effects of these drugs at the early stages of the inflammatory response. Inhibition of cytokine synthesis in the activated macrophages was directly related with the concentration of benzalkonium chloride. Formulation 1 exhibited the most expressive anti-inflammatory effects.

### Materials and methods of antiseptic testing of formulations 4 to 6 in vitro

The study of antiseptic activity of the formulations 4 to 6 was conducted in the testing cultures of reference strains of Haemophilus influenzae (H. influenzae) ATCC 49247, Moraxella catarrhalis ATCC 25235, Staphylococcus aureus (S. aureus) ATCC 29213, purchased from L.A. Tarasevich State Institute of Standardization and Control of Medicinals and Biologicals , as well as using the testing strains of Streptococcus pneumoniae (S. pneumoniae) #1, Streptococcus pyogenes (S. pyogenes) #664, obtained from the clinical materials (Museum of Interclinical Bacteriology Laboratory of I.M. Sechenov First Moscow State Medical University).

The antiseptic activity of the formulations was explored in the liquid growth media and in the solid media.

### The study of antibacterial activity of the formulations 4 to 6 in the liquid growth media

The growth of M. catarrhalis, S. aureus, S. pneumoniae, S. pyogenes occurred on the agar with heart-brain extraction with addition of 5% blood. The growth of H. influenzae was performed in the chocolate agar on the basis of heart-brain extraction. The daily agar culture of the respective microorganism was rinsed with sterile saline, and the resulting bacterial suspension was brought up to the density of 1-1.5 bln of microbial cells per 1 mL, according to McFarland standard. The ready suspensions were used to prepare 10-fold broth dilutions, containing approximately 1.0-1.5x10⁸ CFU/mL of M. catarrhalis, S. pneumoniae, S. pyogenes, H. influenzae - in the brain heart broth, S. aureus - in Mueller-Hinton broth.

The ready broth cultures of the testing microorganisms were dispensed by 4 1-mL tubes. The respective formulations (1 mL) were introduced into the corresponding tubes No.1, No.2, No.3. The tube No.4 served as control tube and contained 1 mL of the respective broth instead of the study formulations. The samples were incubated in the thermostat at t=350°C; the solid medium were inoculated 1 hour and 4 hours apart to measure bacterial density (BD). After 24 to 48 hours of incubation of the inoculated plates, dependent on the type of the microbes, the number of colonies and BD were measured.

### The study of antibacterial activity of the formulations 4 to 6 in the liquid growth media

Standard bacterial suspensions with respective density 0.5, according to McFarland standard (approximately 1.5x10⁸ CFU/mL), were prepared of the daily cultures of tested microbes grown in the respective solid media (chocolate and 5% blood agar with brain heart extraction). The suspension was inoculated per Petri dishes with the use of sterile cotton pad to measure the drug susceptibility. Following 5-minute drying, 1 drop (20 mcL) of each of the formulations 4 to 6 was applied on the surface of the growth media with tested microorganisms. Reading of the results was performed after 24- to 48-hour incubation of the inoculated dishes based on the growth restriction areas of tested microorganisms.

### Results

The results of antimicrobial activity of the formulations 4 to 6 are presented in Table 1 to Table 6.

Table 1 to Table 5 demonstrate significant antiseptic activity of the formulations 4 to 6 against tested bacteria in the liquid media. The formulations 4 to 6 provided elimination of S. aureus, M. catarrhalis, S. pneumoniae, S. pyogenes, H. influenzae within 60 minutes.

**Table 1. Analysis of antiseptic activity of the formulations 4 to 6 against S. aureus ATCC 29213.**

| | BD of tested microorganism (CFU/mL) | | |
|---|---|---|---|
| | Exposure | | |
| | 0 hour | 1 hour | 4 hour |
| Formulation #4 | 2.0 x 10⁸ | No growth | No growth |
| Formulation #5 | 2.0 x 10⁸ | No growth | No growth |
| Formulation #6 | 2.0 x 10⁸ | No growth | No growth |
| Control | 2.0 x 10⁸ | 1 x 10⁸ | 2.0 x 10⁹ |

**Table 2. Analysis of antiseptic activity of the formulations 4 to 6 against H. influenzae ATCC 49247.**

| | BD of tested microorganism (CFU/mL) | | |
|---|---|---|---|
| | Exposure | | |
| | 0 hour | 1 hour | 4 hour |
| Formulation #4 | 1.2 x 10⁸ | No growth | No growth |
| Formulation #5 | 1.2 x 10⁸ | No growth | No growth |
| Formulation #6 | 1.2 x 10⁸ | No growth | No growth |
| Control | 1.2 x 10⁸ | 1.0 x 10⁸ | 1.0 x 10⁸ |

**Table 3. Analysis of antiseptic activity of the formulations 4 to 6 against M. catarrhalis ATCC 25235.**

| | BD of tested microorganism (CFU/mL) | | |
|---|---|---|---|
| | Exposure | | |
| | 0 hour | 1 hour | 4 hour |
| Formulation #4 | 1.4 x 10⁸ | No growth | No growth |
| Formulation #5 | 1.4 x 10⁸ | No growth | No growth |
| Formulation #6 | 1.4 x 10⁸ | No growth | No growth |
| Control | 1.4 x 10⁸ | 1.0 x 10⁷ | 8.0 x 10⁶ |

**Table 4. Analysis of antiseptic activity of the formulations 4 to 6 against S. pyogenes #664.**

| | BD of tested microorganism (CFU/mL) | | |
|---|---|---|---|
| | Exposure | | |
| | 0 hour | 1 hour | 4 hour |
| Formulation #4 | 2.0 x 10⁷ | No growth | No growth |
| Formulation #5 | 2.0 x 10⁷ | No growth | No growth |
| Formulation #6 | 2.0 x 10⁷ | No growth | No growth |
| Control | 2.0 x 10⁷ | 4.0 x 10⁷ | 4.0 x 10⁸ |

**Table 5. Analysis of antiseptic activity of the formulations 4 to 6 against S. pneumoniae #1.**

| | BD of tested microorganism (CFU/mL) | | |
|---|---|---|---|
| | Exposure | | |
| | 0 hour | 1 hour | 4 hour |
| Formulation #4 | 2.0 x 10⁶ | No growth | No growth |
| Formulation #5 | 2.0 x 10⁶ | No growth | No growth |
| Formulation #6 | 2.0 x 10⁶ | No growth | No growth |
| Control | 2.0 x 10⁶ | 2.0 x 10⁶ | 8.0 x 10⁶ |

Moreover, formulations 4 to 6 demonstrated a significant antiseptic activity against tested microorganisms in the studies conducted in solid growth media (Table 6). Within the area of exposure (drop area) of the formulations 4 to 6, a complete growth inhibition of S. aureus, M. catarrhalis, S. pneumoniae, S. pyogenes, H. influenzae was observed.

**Table 6. Antiseptic activities of the formulations 4 to 6**

| Tested microorganism | Present (+) or absent (-) microbial growth | | |
|---|---|---|---|
| | Formulation | | |
| | 4 | 5 | 6 |
| S. aureus | - | - | - |
| M. catarrhalis | - | - | - |
| H. influenzae | - | - | - |
| S. pyogenes | - | - | - |
| S. pneumoniae | - | - | - |

Thus, the formulations 4 to 6 provided significant antiseptic activity against S. aureus, M. catarrhalis, S. pneumoniae, S. pyogenes, and H. influenzae.

Taking into account the difference between the formulations 4 to 6 vs. corresponding formulations 1 to 3, consisting in the use of propyphenazone instead of phenazone, we may conclude that antiseptic and anti-inflammatory properties of two types of formulations are identical, as far as these effects are predominantly associated with the presence and concentration of benzalkonium chloride.

Meanwhile, analgesic and anaesthetic effects, confirmed by less pronounced reactions of rabbits to the exposure of the scarified experimental ear, are related with phenazone and propyphenazone and articaine.

### Preferable formulations

Besides phenazone (propyphenazone), articaine and benzalkonium chloride, the preferable formulations additionally contain the following excipients. These formulations include:
Formulation 7 - phenazone 4% w/w, articaine 2% w/w, benzalkonium chloride 0.2% w/w; additional substance - glycerol;
Formulation 8 - phenazone 4% w/w, articaine 2% w/w, benzalkonium chloride 0.1% w/w; additional substance - glycerol;
Formulation 9 - phenazone 4% w/w, articaine 2% w/w, benzalkonium chloride 0.05% w/w; additional substance - glycerol;
Formulation 10 - propyphenazone 4% w/w, articaine 2% w/w, benzalkonium chloride 0.2% w/w; additional substances - propylene glycol and glycerol;
Formulation 11 - propyphenazone 4% w/w, articaine 2% w/w, benzalkonium chloride 0.1% w/w; additional substances - propylene glycol and glycerol;
Formulation 12 - propyphenazone 4% w/w, articaine 2% w/w, benzalkonium chloride 0.05% w/w; additional substances - propylene glycol and glycerol.

The content of propylene glycol in the above mentioned formulations should be within 40% to 60% w/w. The content of glycerol is about 20% to 30% w/w. The remaining content is represented by water.

These preferable formulations can be produced as follows. Formulations 7 to 9, containing phenazone, were prepared through dissolution of phenazone, articaine and benzalkonium chloride in water at 30-45°C while stirring until complete dissolution, with subsequent adding of glycerol to the resulting mixed solution, stirring until homogeneity of glycerol-containing solution, and subsequent adding of water up to the needed volume, while stirring. Herein, the stirring can be intense, i.e. stirring that could produce the homogeneous solution. Adding of glycerol to the solution obtained as a result of mixing comprise stirring for not more than 30 minutes. Therein, after the gradual adding of water up to the needed volume, filtration can be achieved, which comprises sieve filtration, including nylon sieve. Meanwhile, the formulations 10 to 12, containing propyphenazone, were prepared through dissolution of propyphenazone in propylene glycol at 30-45°C, while stirring until complete dissolution, and dissolution of articaine and benzalkonium chloride in water at 30-45°C, while stirring until complete dissolution, thereafter the resulting solution of propyphenazone in propylene glycol and solution of articaine and benzalkonium chloride in water were mixed with agitation until homogeneity of the resulting solution, after which glycerol was added to the mixed solution until homogeneity of glycerol-based solution, after which water was added up to the needed volume, similarly while stirring. Stirring should be intense, so as to provide homogeneity of the resulting solution. Therein, mixing of the resulting solution of propyphenazone plus propylene glycol with the solution of articaine, benzalkonium chloride and water can be achieved through agitation for not more than 30 minutes. Adding of glycerol to the solution obtained through mixing, can be similarly achieved through stirring for not more than 30 minutes. Therein, after the gradual adding of water up to the needed volume, filtration can be achieved, which comprises sieve filtration, including nylon sieve.

## Claims

1. A pharmaceutical composition that provides treatment of otitis and other inflammatory ear diseases, containing at least phenazone or propyphenazone, and additionally containing articaine and benzalkonium chloride.

2. A pharmaceutical composition according to claim 1, wherein phenazone content is 4% w/w, articaine content is 2% w/w, benzalkonium chloride content is 0.05% to 0.2% w/w, the remaining substances including excipients and water.

3. A pharmaceutical composition according to any of claims 1 or 2, wherein glycerol is one of the excipients.

4. A pharmaceutical composition according to claim 3, wherein glycerol content is 20% to 30% w/w.

5. A pharmaceutical composition according to any of claims 1, 2 or 4, wherein when containing propyphenazone, contains propylene glycol as excipient.

6. A pharmaceutical composition according to claim 5, wherein propylene glycol content is 44% to 60 % w/w.

7. A pharmaceutical composition according to claim 3, wherein when containing propyphenazone, contains propylene glycol as excipient.

8. A pharmaceutical composition according to claim 7, wherein propylene glycol content is 44% to 60 % w/w.

9. A pharmaceutical composition according to any of claims 1, 2, 4. 6 or 7, wherein represents ear drops.

10. A pharmaceutical composition according to claim 3, wherein representing ear drops.

11. A pharmaceutical composition according to claim 5, wherein representing ear drops.

12. A method of production of the pharmaceutical composition aimed to treat otitis and other inflammatory ear diseases, containing, at least, phenazone, articaine and benzalkonium chloride, comprising:
dissolution of phenazone, articaine and benzalkonium chloride in water at 30-45°C, while stirring until complete dissolution;
subsequent adding of glycerol to the solution prepared by mixing, while stirring until homogeneity of the solution obtained through glycerol adding; and
subsequent adding of water up to the needed volume, while stirring.

13. A method of production, according to claim 12, wherein adding of glycerol to the solution obtained through stirring, is executed while stirring within 30 minutes.

14. A method of production according to any of claims 12 or 13, wherein stirring is intense.

15. A method of production according to any of claims 12 or 13, wherein filtration is performed following gradual adding of water up to the given volume.

16. A method of production according to claim 15, wherein filtration comprises sieve filtration.

17. A method of production according to claim 14, wherein filtration is performed following gradual adding of water up to the given volume.

18. A method of production according to claim 17, wherein filtration comprises sieve filtration.

19. A method of production of the pharmaceutical composition aimed to treat otitis and other inflammatory ear diseases, containing, at least, propyphenazone, articaine and benzalkonium chloride, comprising:
dissolution of propyphenazone in propylene glycol at 30-45°C while stirring until complete dissolution, dissolution of articaine and benzalkonium chloride in water at 30-45°C, while stirring until complete dissolution;
subsequent combining of the resulting solutions of propyphenazone plus propylene glycol with the resulting solution of articaine, benzalkonium chloride and water, while stirring until homogeneity of the solution prepared by mixing;
subsequent adding of glycerol to the solution prepared by mixing, while stirring until homogeneity of the solution obtained through glycerol adding; and
subsequent adding of water up to the needed volume, while stirring.

20. A method of production according to claim 19, wherein mixing of the solution of propyphenazone with propylene glycol to the resulting solution of articaine, benzalkonium chloride and water, is executed while stirring within 30 minutes.

21. A method of production according to any of claims 19 or 20, wherein adding of glycerol to the solution obtained through mixing, is executed while stirring within 30 minutes.

22. A method of production according to any of claims 19 or 20, wherein stirring is intense.

23. A method of production according to claim 21, wherein stirring is intense.

24. A method of production according to any of claims 19, 20 or 23, wherein filtration is performed following gradual adding of water up to the given volume.

25. A method of production according to claim 24, wherein filtration comprises sieve filtration.

26. A method of production according to claim 22, wherein filtration is performed following gradual adding of water up to the given volume.

27. A method of production according to claim 26, wherein filtration comprises sieve filtration.

28. A use of pharmaceutical composition according to any of claims 1-11 to treat otitis and other inflammatory ear diseases.

29. A use according to claim 28, wherein otitis or other inflammatory ear diseases comprise outer otitis or middle otitis.

30. A use of pharmaceutical composition produced through the method of production according to any of claims 12-27, to treat otitis and other inflammatory ear diseases.

31. A use according to claim 30, wherein otitis or other inflammatory ear diseases comprise outer otitis or middle otitis.

32. A combined use of phenazone or propyphenazone, and articaine and benzalkonium chloride, to produce pharmaceutical composition aimed to treat otitis and other inflammatory ear diseases.

33. A use according to claim 32, wherein otitis or other inflammatory ear diseases comprise outer otitis or middle otitis.

34. A combined use of phenazone, articaine and benzalkonium chloride to produce pharmaceutical composition aimed to treat otitis and other inflammatory ear diseases.

35. A use according to claim 34, wherein otitis or other inflammatory ear diseases comprise outer otitis or middle otitis.

36. A combined use of propyphenazone, articaine, benzalkonium chloride, propylene glycol and glycerol to produce pharmaceutical composition aimed to treat otitis and other inflammatory ear diseases.

37. A use according to claim 36, wherein otitis or other inflammatory ear diseases comprise outer otitis or middle otitis.

38. A combined use of propyphenazone and articaine to produce pharmaceutical composition for external or local application.

39. A use according to claim 38, wherein pharmaceutical composition comprises ear drops.

40. A method of treatment of otitis and other inflammatory ear diseases, comprising instillation of the pharmaceutical composition according to any of claims 1 to 11, into the ear of patient with inflammatory ear disease.

41. A method of treatment according to claim 40, wherein inflammatory ear diseases comprises outer otitis or middle otitis.

42. A method of treatment of otitis and other inflammatory ear diseases, comprising instillation of the pharmaceutical composition produced according to any of claims 12 to 27, into the ear of patient with inflammatory ear disease.

43. A method of treatment according to claim 42, wherein inflammatory ear diseases comprises outer otitis or middle otitis.
